# EUROPÄISCHE PATENTSCHRIFT

(11) **EP 3 507 274 B1**
(45) Veröffentlichungstag und Bekanntmachung des Hinweises auf die Patenterteilung: **12.08.2020**
(21) Anmeldenummer: 17758108.9
(22) Anmeldetag: 17.08.2017
(51) Int. Cl.: C07D 207/16, C12P 13/22

(54) **VERFAHREN ZUR HERSTELLUNG CHIRALER AMINONITRILE**
METHOD FOR PRODUCING CHIRAL AMINONITRILES
PROCÉDÉ DE PRÉPARATION D'AMINONITRILES CHIRAUX

(30) Priorität: 30.08.2016 DE 102016116130
(43) Veröffentlichungstag der Anmeldung: 10.07.2019
(73) Patentinhaber: Universität Bielefeld, 33615 Bielefeld (DE)
(72) Erfinder: GRÖGER, Harald, 33739 Bielefeld (DE); BETKE, Tobias, 33611 Bielefeld (DE); ROMMELMANN, Philipp, 33607 Bielefeld (DE)
(74) Vertreter: Michalski Hüttermann & Partner Patentanwälte mbB
(86) Internationale Anmeldenummer: PCT/EP2017/070820
(87) Internationale Veröffentlichungsnummer: WO 2018/041639

(56) Entgegenhaltungen:
- EP-A1- 0 536 399
- EP-A1- 1 211 246
- WO-A1-00/34241
- WO-A1-90/12005
- WO-A1-2010/051188
- WO-A2-2004/052850
- WO-A2-2007/147397
- LAURENT PELLEGATTI ET AL: "Synthesis of Vildagliptin Utilizing Continuous Flow and Batch Technologies", ORGANIC PROCESS RESEARCH AND DEVELOPMENT, Bd. 19, Nr. 4, 17. April 2015 (2015-04-17) , Seiten 551-554, XP55417513, US ISSN: 1083-6160, DOI: 10.1021/acs.oprd.5b00058 in der Anmeldung erwähnt

## Beschreibung

Die Erfindung betrifft das Gebiet der organischen Synthese, insbesondere ein Verfahren zur Herstellung chiraler *N*-Acyl- und *N*-Sulfonyl-α-aminonitrile.

Enantiomerenangereicherte, insbesondere enantiomerenreine, *N*-Acyl-α-aminonitrile vom (*R*)- und (*S*)-Typ sind wertvolle Synthesebausteine bei der Herstellung moderner Arzneistoffe mit einer chiralen Nitril-Einheit, beziehungsweise stellen solche Arzneistoffe dar. Beispiele für solche pharmazeutischen Wirkstoffe sind Gliptine wie Vildagliptin und Saxagliptin, sowie NVP-DPP-728. Gliptine wirken als Dipeptidylpeptidase-4-Inhibitoren und werden als Arzneimittel zur Behandlung von Diabetes mellitus Typ 2 eingesetzt. Der Wirkstoff Vildagliptin wurde von der Firma Novartis entwickelt und 2013 mit einem Umsatzvolumen von 1,2 Milliarden US-Dollar gegen Diabetes Typ II vermarktet. In der Schrift WO 2000 034 241 A ist ein Verfahren zu dessen Herstellung beschrieben. Saxagliptin sowie ein Verfahren zu dessen Herstellung ist in der Schrift WO 2004 052 850 A beschrieben.

Eine wichtige Zwischenstufe bei der Synthese dieser Gliptine sind enantiomerenreine N-geschützte oder *N*-acylierte Pyrrolidin-2-nitrilderivate. Typischerweise erfolgt der Zugang zu *N*-acylierten chiralen Nitrilen vom (*R*)- und (*S*)-Typ immer noch durch Mehrstufensynthesen. Nachteilig bei den im Stand der Technik bekannten Synthesezugängen zu enantiomerenreinen *N*-Acyl-α-aminonitrilen ist insbesondere, dass diese auf dem Einsatz hochtoxischer Cyanide oder anderer toxischer Reagenzien wie dem Vilsmeier-Reagenz beruhen. Zudem kommen bei dessen Herstellung bereits toxische Reagenzien wie Oxalylchlorid bzw. Phosphoroxychlorid zum Einsatz.

So basiert die Herstellung der α-Aminonitrile, die *via* Strecker-Reaktion gut zugänglich sind, wie die der meisten bekannten Verfahren zur Herstellung von chiralen, enantiomerenangereicherten bzw. enantiomerenreinen Nitrilen auf dem Einsatz von hochtoxischen Cyaniden. Zur Stabilisierung dieser generell eher labilen und zur Rückreaktion unter Freisetzung von hochtoxischer Blausäure neigenden Verbindungen werden diese vorzugsweise acyliert. Allerdings werden diese Synthesen typischerweise mit acyclischen Iminen durchgeführt, wobei hierbei weder ein direkter Synthesezugang zu Prolin-analogen Nitrilen noch zu α-Aminonitrilen mit einer primären Aminogruppe als Nitril-Analoga der acyclischen proteinogenen α-Aminosäuren erreicht wird. Aus der Perspektive der Chemikalien- und Prozesssicherheit sowie der Nachhaltigkeit und Umweltverträglichkeit eines chemischen Produktionsverfahrens sind Cyanid-freie Routen zu Nitrilen von großem Interesse.

Eine bekannte und industriell angewandte Alternative zur Herstellung von aus Aminosäuren abgeleiteten Nitrilen stellen Derivatisierungsmethoden ausgehend von enantiomerenreinen Aminosäuren dar. Hierbei wird die Aminosäure zunächst in ein Amid überführt, bevor anschließend dieses Amid aktiviert und in das gewünschte Nitril umgewandelt wird. Dieser Syntheseansatz, der auf dem Einsatz eines Vilsmeier-Reagenzes sowie auf dem Konzept der "chiral-Pool-Derivatisierung" basiert, kommt beispielsweise bei der Synthese von Vildagliptin zum Einsatz, wie von L. Pellegatti und J. Sedelmeier in Org. Process Res. Dev., 2015, 19, S. 551-554 beschrieben. Nachteilig ist bei diesem Verfahren jedoch, dass zum einen zunächst das Amid in aufwändiger Weise aus L-Prolin synthetisiert werden muss. Gerade die Herstellung nicht-substituierter Amide unter Einsatz von lediglich Ammoniak ist nicht trivial. Zum anderen muss das sogenannte "Vilsmeier-Reagenz" in aufwändiger und mit einer hohen Abfallmenge verbundenen Weise hergestellt werden. Daher besteht ein Bedarf an alternativen Synthesemethoden chiraler α-Aminonitrile.

WO 90/12005 A1 beschreibt Aminosäure-Derivate mit Prolylendopeptid-Inhibitorwirkung. EP 0 536 399 A1 beschreibt Arylalkanoylaminderivate und diese enthaltende Arzneimittel. EP 1 211 246 A1 beschreibt Pyrimidinderivate und Herbizide, welche diese enthalten. WO 2010/051188 A1 beschreibt P2X3-Rezeptorantagonisten zur Schmerzbehandlung.

Der vorliegenden Erfindung lag daher die Aufgabe zu Grunde, ein Verfahren zur Verfügung zu stellen, dass mindestens einen der vorgenannten Nachteile des Standes der Technik überwindet. Insbesondere lag der vorliegenden Erfindung die Aufgabe zu Grunde, ein Verfahren bereit zu stellen, das die Herstellung chiraler α-Aminonitrile unabhängig von hochtoxischen Cyaniden und problematischen Reagenzien wie dem Vilsmeier-Reagenz erlaubt.

Diese Aufgabe wird gelöst durch ein Verfahren zur Herstellung eines *N*-Acyl- oder *N-*Sulfonyl-α-aminonitrils gemäß Anspruch 1.

Das erfindungsgemäße Verfahren erlaubt ausgehend von gut zugänglichen *N*-Acyl-α-Aminoaldehyden oder *N*-Sulfonyl-α-aminoaldehyden als Substratkomponente und der Umwandlung der Aldehyd-Komponente in eine Aldoxim-Einheit *via* Kondensation mit Hydroxylamin sowie anschließender Dehydratisierung der Aldoxim-Einheit zum Nitril die Herstellung chiraler *N*-Acyl- oder *N*-Sulfonyl-α-aminonitrile in präparativ einfacher und wirtschaftlicher Weise. Überraschend wurde gefunden, dass während dieses Syntheseprozesses die Enantiomerenreinheit erhalten bleibt bzw. nur in geringfügiger Menge vermindert wird und somit die sonst bei solchen zur Enolbildung befähigten Verbindungsklassen typischerweise beobachtbare Racemisierung via Keto-Enol-Tautomerie unterbunden werden kann. Das Verfahren ist insbesondere für die Herstellung von enantiomerenangereicherten und vorzugsweise von enantiomerenreinen *N*-Acyl- oder *N-*Sulfonyl-α-aminonitrilen geeignet.

Das Verfahren verwendet als Edukt *N*-Acyl-oder *N*-Sulfonyl-α-Aminoaldehyde, die ausgehend von α-Aminosäuren durch *N*-Acylierung und Umwandlung der Carbonsäure-Funktion in eine Aldehydfunktion, in vorteilhafter Weise in enantiomerenangereicherter, insbesondere enantiomerenreiner Form, leicht erhältlich sind. Hierdurch stellt das Verfahren vielfältige Vorteile zur Verfügung. Basierend auf gut zugänglichen Aminosäuren und Hydroxylamin als Bulkchemikalie sind Aldoxime als Substrat einfach zugänglich. Weiterhin sind die Reaktionsschritte robust gegenüber Racemisierung. Von Vorteil ist weiter, dass das Verfahren keinen Einsatz von hochtoxischem Cyanid bzw. aufwändig zu synthetisierenden und mit erheblichen Abfallmengen verbundenen Vilsmeier-Reagenz benötigt. So ist das Verfahren präparativ einfach durchführbar und zeichnet sich durch eine hohe Praktikabilität aus. Insgesamt erlaubt das Verfahren in vorteilhafter Weise die Herstellung der gewünschten *N*-Acyl-und *N*-Sulfonyl-α-aminonitrile ausgehend von einfach zugänglichen und kostenattraktiven Ausgangsverbindungen unter sanften Reaktionsbedingungen ohne den Einsatz problematischer Reagenzien. Zudem sind hohe Umsätze, hohe Ausbeuten und exzellente Enantiomerenüberschüsse erzielbar.

Das Verfahren ist zur Herstellung chiraler *N*-Acyl- und *N*-Sulfonyl-α-aminonitrile geeignet. Unter dem Begriff "chiral" wird im Sinne der vorliegenden Erfindung eine Verbindung verstanden, die wenigstens ein Stereozentrum aufweist, dessen Substituenten ihre relative Lage zueinander nicht ändern können. Hierdurch sind verschiedene räumliche Anordnungen möglich. Dies ist beispielsweise der Fall, wenn in einem Molekül ein Kohlenstoffatom vier verschiedene Substituenten trägt. Dieses Kohlenstoffatom wird als Stereozentrum oder Chiralitätszentrum bezeichnet. In bevorzugten Ausführungsformen setzt man in Schritt a) ein enantiomerenangereichertes oder enantiomerenreines *N*-Acyl- oder *N*-Sulfonyl-α-aminoaldehyd ein. Ein großer Vorteil des Verfahrens ist, dass dessen absolute Konfiguration bei der Umwandlung zum *N*-Acyl- oder *N*-Sulfonyl-α-aminonitril erhalten oder im Wesentlichen erhalten bleibt. Unter der Formulierung, dass die absolute Konfiguration im Wesentlichen erhalten bleibt ist zu verstehen, dass der Enantiomerenüberschuss, oder kurz ee-Wert (englisch: enantiomeric excess) sich leicht vermindern kann, beispielsweise von ≥ 99% ee auf ≥ 95% oder ≥ 90% ee.

Das Verfahren umfasst die folgenden Schritte:
a) Kondensation eines *N*-Acyl- oder *N*-Sulfonyl-α-aminoaldehyds gemäß der allgemeinen Formel (I) mit Hydroxylamin zu einem Aldoxim gemäß der allgemeinen Formel (II), und
b) Dehydratisierung des in Schritt a) erhaltenen Aldoxims zu einem N-Acyl- oder *N-*Sulfonyl-α-aminonitril gemäß der allgemeinen Formel (III): 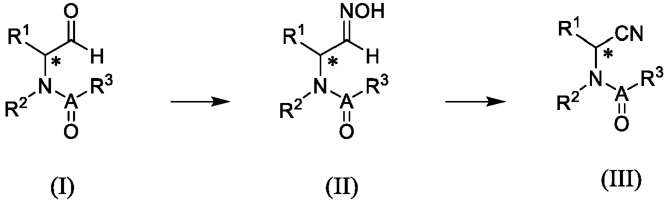 worin:
   - A: ist C oder S=O;
   - R¹: ist ausgewählt aus der Gruppe umfassend verzweigtes oder unverzweigtes C₁-C₂₀-Alkyl, C₆-C₁₀-Aryl, C₆-C₁₆-Heteroaryl, C₇-C₁₆-Arylalkyl und/oder C₆-C₁₆-Heteroarylalkyl, wobei diese unsubstituiert oder einfach oder mehrfach substituiert mit wenigstens einem Substituenten ausgewählt aus der Gruppe umfassend OH, NH₂, NHR⁴, NR⁴₂, C₁₋₄-Alkyl, C₇-C₁₆-Arylalkyl, C₆-C₁₆-Heteroarylalkyl, Carbonylsauerstoff und/oder C₁₋₄-Alkoxy sind;
   - R²: ist ausgewählt aus der Gruppe umfassend H, verzweigtes oder unverzweigtes C₁-C₂₀-Alkyl, C₆-C₁₀-Aryl, C₆-C₁₆-Heteroaryl, C₇-C₁₆-Arylalkyl und/oder C₆-C₁₆-Heteroarylalkyl, wobei diese unsubstituiert oder einfach oder mehrfach substituiert mit wenigstens einem Substituenten ausgewählt aus der Gruppe umfassend OH, NH₂, NHR⁴, NR⁴₂, C₁₋₄-Alkyl, C₇-C₁₆-Arylalkyl, C₆-C₁₆-Heteroarylalkyl, Carbonylsauerstoff und/oder C₁₋₄-Alkoxy sind; oder
   R¹ und R² bilden gemeinsam einen gesättigten 5- oder 6-gliedrigen Ring oder ein bicyclisches Ringsystem aus, wobei diese wenigstens ein weiteres Heteroatom ausgewählt aus N, O und/oder S enthalten können und/oder diese einfach oder mehrfach mit wenigstens einem Substituenten ausgewählt aus der Gruppe umfassend OH, NH₂, NHR⁴, NR⁴₂, C₁₋₄-Alkyl, Carbonylsauerstoff und/oder C₁₋₄-Alkoxy substituiert sein können;
   R³ ist ausgewählt aus der Gruppe umfassend H, C₁₋₆-Alkoxy und/oder C₁₋₆-Alkyl, wobei diese einfach oder mehrfach substituiert sind mit wenigstens einem Substituenten ausgewählt aus der Gruppe umfassend OH, OR⁴, NH₂, NHR⁴, NR⁴₂, NHY und/oder Halogen; oder ist ausgewählt aus der Gruppe der Strukturelemente (IV), (V) und (VI) wie folgt: 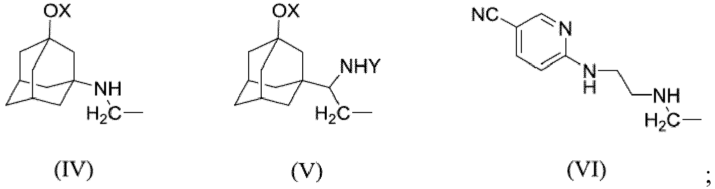
   R⁴ ist jeweils gleich oder unabhängig voneinander ausgewählt aus der Gruppe umfassend C₁-C₁₈-Alkyl oder C₁-C₁₈-Acyl;
   X, Y sind jeweils gleich oder unabhängig voneinander H oder eine Schutzgruppe insbesondere ausgewählt aus *tert*-Butyloxycarbonyl, Benzyloxycarbonyl, Acetyl, Silyl, p-Tolyl, Trifluormethyl und/oder Sulfonyl.

Der Begriff "C₁-C₂₀-Alkyl" umfasst, wenn nicht anders angegeben, geradkettige oder verzweigte Alkylgruppen mit 1 bis 20 Kohlenstoffatomen. Alkylgruppen sind vorzugsweise ausgewählt aus der Gruppe umfassend Methyl, Ethyl, Propyl, Isopropyl, Butyl, Isobutyl, Pentyl, Isopentyl, Neopentyl, Hexyl, Isohexyl Heptyl, Isoheptyl, Octyl, Isooctyl, 2-Ethylhexyl, Neooctyl, Nonyl, Isononyl, Neononyl, Decyl, Isodecyl und/oder Neodecyl. Bevorzugt sind C₁-C₆-Alkyl-Gruppen ausgewählt aus der Gruppe umfassend Methyl, Ethyl, Propyl, Isopropyl, Butyl und/oder *tert*-Butyl.

Unter dem Begriff "Aryl" sind aromatische Reste mit 6 bis 10 Kohlenstoff-Atomen zu verstehen. Der Begriff "Aryl" umfasst bevorzugt Carbocyclen, insbesondere Phenyl.

Im Sinne der vorliegenden Erfindung ist der Begriff "Arylalkyl" so zu verstehen, dass dieser über den Alkylteil gebunden ist. Der Arylteil kann 6 bis 10 Kohlenstoffatome aufweisen und der Alkylteil 1 bis 6 Kohlenstoffatome, bevorzugt ist Phenylalkyl mit 1 bis 4 Kohlenstoffatomen im Alkylteil, insbesondere Benzyl.

C₁-C₆-Alkoxygruppen sind vorzugsweise ausgewählt aus der Gruppe umfassend Methoxy, Ethoxy, lineares oder verzweigtes Propoxy und/oder Butoxy.

Im Sinne der vorliegenden Erfindung sind, wenn nicht abweichend angegeben, unter dem Begriff "Heteroaryl" mono-, bi- oder tricyclische Heteroarylgruppen umfassend ein, zwei, drei oder vier Heteroatome ausgewählt aus der Gruppe umfassend N, O und/oder S zu verstehen. Bevorzugte Heteroarylgruppen sind monocyclische Heteroarylgruppen. Bevorzugte monocyclische Heteroarylgruppen weisen ein Heteroatom auf. Bevorzugte Heterocyclylgruppen sind ausgewählt aus der Gruppe umfassend Furanyl, Pyrrolyl, Pyridinyl, und/oder Thienyl. Besonders bevorzugte Heteroarylgruppen sind ausgewählt aus der Gruppe umfassend Furanyl und/oder Thienyl.

Der Begriff "C₁-C₁₈-Acyl" umfasst im Rahmen der Erfindung vorzugsweise geradkettige oder verzweigte Acylreste mit 1 bis 18 Kohlenstoffatomen. Bevorzugte C₁-C₁₀-Acyl-Gruppen sind ausgewählt aus der Gruppe umfassend Formyl, Acetyl, Propanoyl, Isopropanoyl, Butanoyl, Isobutanoyl, Pentanoyl und/oder Isopentanoyl. Bevorzugt ist ein geradkettiger oder verzweigter C₁-C₄-Acylrest. Besonders bevorzugt ist Acetyl.

Der Begriff "Halogen" umfasst Fluor, Chlor, Brom und Jod, wobei Fluor oder Chlor insbesondere Chlor bevorzugt sind.

Der Begriff "Schutzgruppe" beschreibt im Rahmen der Erfindung einen Substituenten, der während der Synthese eingeführt wird, um eine funktionelle Gruppe beispielsweise eine Hydroxylgruppe vorübergehend zu schützen und unerwünschte Reaktionen zu verhindern. Die Schutzgruppe kann im Rahmen des Verfahrens wieder abgespalten werden oder am N-Acyl- oder *N*-Sulfonyl-α-aminonitril verbleiben, beispielsweise wenn dieses für weitere Syntheseschritte verwendet werden soll. Bevorzugte Schutzgruppen sind ausgewählt aus *tert-*Butyloxycarbonyl (Boc), Benzyloxycarbonyl, Acetyl, Silyl, p-Tolyl, Trifluormethyl und/oder Sulfonyl. Bevorzugte Silyl-Schutzgruppen sind ausgewählt aus Trimethylsilyl (TMS), *tert-*Butyldimethylsilyl (TBDMS), Triethylsilyl (TES), *tert*-Butyldiphenhylsilyl (TBDPS) und Triisopropylsilyl (TIPS). Bevorzugte Sulfonyl-Schutzgruppen sind ausgewählt aus p-Toluensulfonat (Tosyl) oder Methylsulfonat (Mesyl). Schutzgruppen sind insbesondere einsetzbar, um *N*-geschützte oder *N*-acylierte Pyrrolidin-2-nitrilderivate zu erhalten, die vorteilhaft in Synthesen der Gliptine verwendbar sind.

In bevorzugten Ausführungsformen ist A ein Kohlenstoffatom. Insbesondere *N*-Acyl-α-aminonitrile sind in vorteilhafter Weise als Synthesebausteine für Arzneistoffe mit chiraler Nitril-Einheit verwendbar oder bilden einen Wirkstoff. Es kann ebenfalls bevorzugt sein, dass A für eine Gruppe S=O steht. Auch chirale *N*-Sulfonyl-α-aminonitrile sind in der Wirkstoffchemie vorteilhaft verwendbar. Insbesondere ist eine Sulfonylgruppe leicht abspaltbar, so dass hierdurch eine primäre oder sekundäre Aminogruppe bereit gestellt werden kann.

Die Substituenten R¹ und R² können jeweils gleich oder unabhängig voneinander verzweigtes oder unverzweigtes C₁-C₅-Alkyl, Phenyl, oder C₇-C₁₀-Phenylalkyl sein. Der Substituent R² kann hierbei auch Wasserstoff sein. In anderen Ausführungsformen können die Substituenten R¹ und R² gemeinsam einen gesättigten 5- oder 6-gliedrigen Ring oder ein bicyclisches Ringsystem ausbilden. Das ausgebildete Ringsystem enthält in diesen Fällen bereits ein Stickstoffatom, kann jedoch noch weitere Heteroatome insbesondere Stickstoff oder Sauerstoff enthalten. Die Substituenten R¹ und R² können jeweils ihrerseits noch substituiert sein, insbesondere mit einer Gruppe ausgewählt aus OH, NH₂, C₁₋₄-Alkyl oder einem Carbonylsauerstoff.

Der Substituent R³ kann Wasserstoff sein, insbesondere für den Fall, dass A ein Kohlenstoffatom ist. Vorzugsweise kommen typische für die Amino-Funktion von Aminosäuren angewendete Schutzgruppen zum Einsatz. Daher ist insbesondere für den Fall, dass A ein Kohlenstoffatom ist, der Substituent R³ vorzugsweise eine C₁₋₅-Alkoxygruppe, insbesondere *tert*-Butoxy, oder eine Halogen- insbesondere Chlor-substituierte C₁₋₃-Alkylgruppe insbesondere Chloromethyl. Insbesondere im Rahmen der Synthese der Gliptine ist der Substituent R³ ein Strukturelement (IV), (V) oder (VI). Insbesondere bei der Synthese der Gliptine sind enantiomerenreine *N*-geschützte oder *N*-acylierte Pyrrolidin-2-nitrilderivate als Produkt des Verfahrens bevorzugt.

Als Substrat verwendbare *N*-Acyl- oder *N*-Sulfonyl-α-aminoaldehyde sind kommerziell erhältlich oder beispielsweise ausgehend von α-Aminosäuren durch *N*-Acylierung und Umwandlung der Carbonsäure-Funktion in eine Aldehydfunktion, leicht erhältlich. Die Substituenten R¹ und R² können in Ausführungsformen daher den Seitenketten von Aminosäuren entsprechen. Insbesondere Phenylalanin und Prolin sind vorteilhaft verwendbare Aminosäuren. In einer sehr bevorzugten Ausführungsform kann R¹ Benzyl sein, während R² Wasserstoff ist. In diesem Fall kann das Substrat ausgehend von der Aminosäure Phenylalanin bereitgestellt werden. In einer weiter sehr bevorzugten Ausführungsform können R¹ und R² gemeinsam einen gesättigten 5-gliedrigen Ring ausbilden. In diesem Fall kann das Substrat ausgehend von der Aminosäure Prolin bereitgestellt werden. L-Prolin ist ein einfach zugänglicher Naturstoff.

In einer bevorzugten Ausführungsform sind die Substituenten der Verbindungen gemäß der allgemeinen Formeln (I), (II) und (III) die folgenden:
- A: ist C;
- R¹: ist ausgewählt aus der Gruppe umfassend Benzyl und/oder C₁-C₂-Alkyl,
- R²: ist ausgewählt aus der Gruppe umfassend H, Benzyl und/oder C₁-C₂-Alkyl, oder
- R¹ und R²: bilden gemeinsam einen gesättigten 5-gliedrigen Ring oder Bicyclo[3.1.0]hexan aus, und
- R³: ist ausgewählt aus der Gruppe umfassend H, *tert*-Butoxy, Chloromethyl, Strukturelemente (IV), (V) und/oder (VI).

In vorteilhafter Weise sind die Reaktionsschritte robust gegenüber Racemisierung. So sind chirale *N*-Acyl- oder *N*-Sulfonyl-α-Aminonitrile mit exzellentem Enantiomerenüberschuss, in enantiomerenangereicherter, insbesondere enantiomerenreiner Form, erzielbar.

Weitere Vorteile ergeben sich daraus, dass das Verfahren die Herstellung von *N*-Acyl- oder *N*-Sulfonyl-α-Aminonitril in präparativ einfacher Form und unter milden Bedingungen erlaubt. Hierdurch ist das Verfahren präparativ einfach und wirtschaftlich durchführbar. Zudem sind hohe Umsätze und hohe Ausbeuten an enantiomerenangereicherten oder enantiomerenreinem Produkt erzielbar.

Die Dehydratisierung in Schritt b) erfolgt vorzugsweise unter Verwendung eines Chemokatalysators. In bevorzugten Ausführungsformen führt man die Dehydratisierung des Aldoxims zum *N*-Acyl- oder *N*-Sulfonyl-α-Aminonitril in Schritt b) in Gegenwart eines Übergangsmetall-Katalysators, insbesondere eines Cu(II)-, Zn(II)-, Co(II)- oder Ni(II)-Katalysators, durch. Hierbei haben sich Cu(II)-basierte Chemokatalysatoren als besonders geeignet erwiesen. Besonders bevorzugt ist Kupfer(II)acetat.

In bevorzugten Ausführungsformen liegt der Stoffmengenanteil des Katalysators im Bereich von ≥ 0,1 mol% bis ≤ 25 mol%, vorzugsweise im Bereich von ≥ 1 mol% bis ≤ 10 mol%, bevorzugt im Bereich von von ≥ 2 mol% bis ≤ 5 mol%. Der Stoffmengenanteil des Katalysators ist hierbei auf die Stoffmenge des Substrats bezogen. Insbesondere wurden gute Ergebnisse bereits bei verwendeten Mengen von 2 mol% Cu(II) als katalytisch aktiver Metallspezies erzielt.

Vorzugsweise führt man die Kondensation des Aldehyds, insbesondere gemäß der allgemeinen Formel (I), in Schritt a) mit Hydroxylamin in wässriger Lösung, insbesondere in einer Mischung aus Wasser und Alkohol durch. Bevorzugte Alkohole sind ausgewählt aus der Gruppe umfassend Methanol, Ethanol, Isopropanol, *n*-Propanol, *n*-Butanol, *tert*-Butanol, Phenol und/oder Mischungen davon. Der Alkohol ist insbesondere ausgewählt aus n-Propanol und/oder Ethanol. Besonders geeignet sind Mischungen von Wasser und Alkohol, beispielsweise Mischungen von Wasser mit Ethanol und/oder n-Propanol. Aus wässriger bzw. alkoholischer Lösung kann das Aldoxim in einfacher Weise isoliert und aufgereinigt werden.

Für die Dehydratisierung des Aldoxims zum Nitril in Schritt b) sind insbesondere organische Lösungsmittel verwendbar. Vorzugsweise führt man die Dehydratisierung des Aldoxims zum *N*-Acyl- oder *N*-Sulfonyl-α-Aminonitril in einem Lösungsmittel ausgewählt aus Dichlormethan, Methyl-*tert*-butylether, Ethylacetat, Tetrahydrofuran, 2-Methyltetrahydrofuran, Toluol, Acetonitril, Propionitril, Butyronitril und/oder deren Mischungen durch. In bevorzugten Ausführungsformen führt man die Dehydratisierung des Aldoxims zum *N*-Acyl- oder *N*-Sulfonyl-α-Aminonitril in Schritt b) in Anwesenheit einer Nitril-Komponente durch. Vorzugsweise ist das Nitril ausgewählt aus der Gruppe umfassend Acetonitril, Propionitril und/oder Butyronitril. Besonders bevorzugt ist Acetonitril. In vorteilhafter Weise bilden diese Nitrile ein gutes und selektives Reagenz zur Überführung des Aldoxims durch Dehydratisierung in die entsprechenden Nitrile. Vorzugsweise liegt die Nitril-Komponente im molaren Überschuss, beispielsweise im Bereich von ≥ 10 Äq. (Äquivalenten) bezogen auf das Aldoxim vor. Hierdurch kann eine Umlagerung zum Amid verhindert oder deutlich unterdrückt werden. Das Stoffmengenverhältnis von Acetonitril zu Aldoxim beträgt vorzugsweise mindestens 10:1. Die Nitril-Komponente kann auch in höherem Anteil beispielsweise von ≥ 15 Äq. bezogen auf das Aldoxim vorliegen. In weiteren Ausführungsformen sind Mischungen eines Nitrils, insbesondere Acetonitril, mit anderen Lösungsmitteln wie Dichlormethan bevorzugt.

Die Kondensation des Aldehyds mit Hydroxylamin in Schritt a) kann bei Umgebungstemperatur erfolgen. Die Dehydratisierung des Aldoxims zum Nitril in Schritt b) erfolgt vorzugsweise bei erhöhten Temperaturen oder unter Rückfluss. In bevorzugten Ausführungsformen führt man die Dehydratisierung des Aldoxims zum *N*-Acyl- oder *N-*Sulfonyl-α-Aminonitril in Schritt b) bei einer Temperatur im Bereich von ≥ 20°C bis ≤ 150°C, vorzugsweise im Bereich von ≥ 50°C bis ≤ 100°C, bevorzugt im Bereich von ≥ 80°C bis ≤ 85°C, durch. Dass die Reaktion bei milden Temperaturen durchgeführt werden kann, vereinfacht die Reaktionsführung deutlich. Es kann vorgesehen sein, dass sich an eine Reaktionszeit von 1 bis 7 oder 8 Stunden bei diesen Temperaturen eine weitere Umsetzungsphase von bis zu 20 Stunden bei Umgebungstemperatur anschließt.

Von besonderem Vorteil ist, dass das Verfahren die Synthese der als Arzneiwirkstoff geeigneten Gliptine vereinfachen kann. So ist das Verfahren insbesondere geeignet für die Herstellung von enantiomerenreinem *N*-geschützten Pyrrolidin-2-nitril, beispielsweise dem *N-*Boc-geschützten Analoga. Dieser Verbindungstyp, der auch als Cyano-Analoga von N-acyliertem L-Prolin aufgefasst werden kann, stellt eine wichtige Zwischenstufe bei der Herstellung des Wirkstoffs Vildagliptin wie auch des Wirkstoffs NVP-DPP-728, dar. Weiter ist das Verfahren vorteilhaft geeignet für die Herstellung *N*-acylierter Pyrrolidin-2-nitrilderivate, die sich als Zwischenstufen für die Herstellung von Saxagliptin eignen.

Ein besonderer Aspekt der Erfindung betrifft ein Verfahren zur Herstellung von Vildagliptin oder dessen Salzen, umfassend die folgenden Schritte:
a) Kondensieren eines Aldehyds der Formel (1) mit Hydroxylamin zu einem Aldoxim der Formel (2): 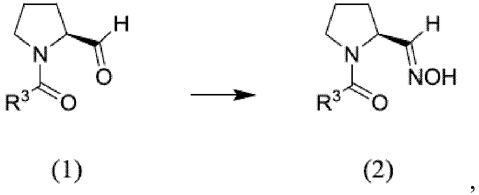 worin
   - R³: ist -CH₂- substituiert mit einem Substituenten ausgewählt aus der Gruppe umfassend OH, OR⁴, NH₂, NHR⁴, NR⁴₂, NHY und/oder Halogen oder Strukturelement (IV) wie folgt: 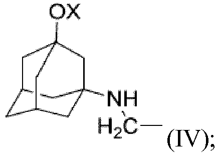
   - X, Y: sind jeweils gleich oder unabhängig voneinander H oder eine Schutzgruppe insbesondere ausgewählt aus *tert*-Butyloxycarbonyl (Boc), Benzyloxycarbonyl, Acetyl, Silyl, p-Tolyl, Trifluormethyl und/oder Sulfonyl:
   - R⁴: ist jeweils gleich oder unabhängig voneinander ausgewählt aus der Gruppe umfassend C₁-C₁₈-Alkyl oder C₁-C₁₈-Acyl;
b) Dehydratisierung des in Schritt a) erhaltenen Aldoxims der Formel (2) zu einem N-Acyl-α-aminonitril der Formel (3): 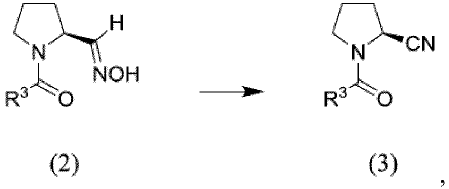
c) optional Umsetzen des *N*-Acyl-α-aminonitrils der Formel (3) mit R³ ist -CH₂-substituiert mit einem Substituenten ausgewählt aus der Gruppe umfassend OH, OR⁴, NH₂, NHR⁴, NR⁴₂, NHY und/oder Halogen mit 1-Aminoadamantan-3-ol oder einem geschützten Derivat der Formel (4) zur Verbindung der Formel (5): 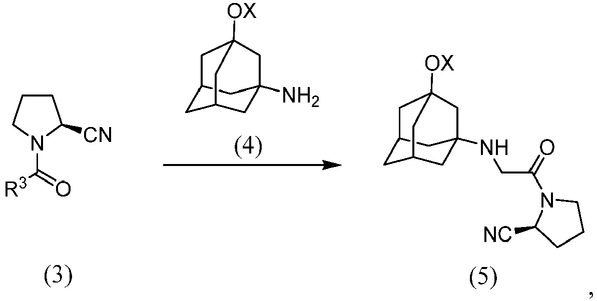 und
d) optional Abspalten der Schutzgruppe X zu Vildagliptin der Formel (6): 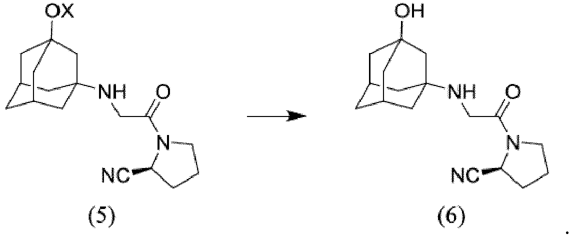 Die Einführung des Adamantylrestes kann bei der Herstellung von Vildagliptin in einem der Herstellung des Nitrils nachgelagerten Schritt erfolgen oder alternativ bereits auf der Oxim-Stufe enthalten sein. Entsprechend kann der Substituent R³ im Aldehyd (1) eine substituierte -CH₂-Gruppe oder das Adamantylelement (IV) sein. Für den Fall, dass der Adamantylrest bereits im Aldehyd (1) enthalten ist, entspricht das *N*-Acyl-α-aminonitril (3) bereits dem gewünschten, ggf. geschützten, Vildagliptin und Schritt c) kann entfallen.

Die Substituenten X und Y sind jeweils Wasserstoff oder eine Schutzgruppe. Insbesondere leicht abspaltbare Acyl-Schutzgruppen wie *tert*-Butyloxycarbonyl (Boc), Acetyl oder Silyl, insbesondere Trimethylsilyl oder -S(O₂)R, insbesondere Tosyl (CH₃-C₆H₄-SO₂-) sind bevorzugt.

Ein weiterer besonderer Aspekt der Erfindung betrifft ein Verfahren zur Herstellung von Saxagliptin oder dessen Salzen, umfassend die folgenden Schritte:
a) Kondensation eines Aldehyds der Formel (7) mit Hydroxylamin zu einem Aldoxim der Formel (8): 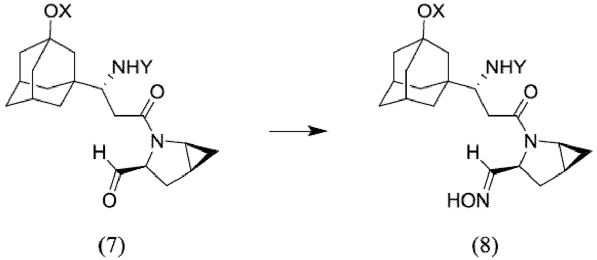 worin:
   - X, Y: sind jeweils gleich oder unabhängig voneinander H oder eine Schutzgruppe insbesondere ausgewählt aus *tert*-Butyloxycarbonyl (Boc), Benzyloxycarbonyl, Acetyl, Silyl, p-Tolyl, Trifluormethyl und/oder Sulfonyl;
b) Dehydratisierung des in Schritt a) erhaltenen Aldoxims der Formel (8) zu einem N-Acyl-α-aminonitril der Formel (9): 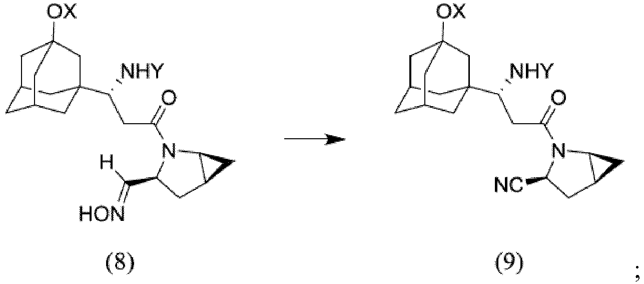
c) optional Abspalten der Schutzgruppen X, Y zu Saxagliptin (10):

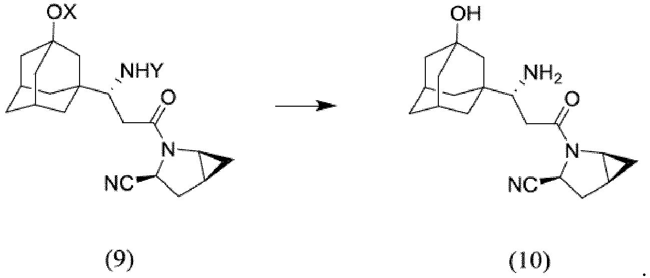

Bei der Synthese von Saxagliptin ist eine nachträgliche Substitution unter Einführung des Adamantyl-Fragments im Unterschied zur Herstellung von Vildagliptin erschwert. Daher ist der Substituent auf der Stufe des Oxims schon enthalten. Die Substituenten X und Y sind jeweils Wasserstoff oder eine Schutzgruppe. Insbesondere leicht abspaltbare Acyl-Schutzgruppen wie *tert*-Butyloxycarbonyl (Boc), Acetyl oder Silyl, insbesondere Trimethylsilyl oder -S(O₂)R, insbesondere Tosyl (CH₃-C₆H₄-SO₂-) sind bevorzugt.

Für die Verfahrensbedingungen der Herstellung von Vildagliptin und Saxagliptin oder deren Salzen wird auf die vorstehende Beschreibung Bezug genommen. Vorteile ergeben sich insbesondere aus der präparativ einfachen Form und den milden Bedingungen. Dies erlaubt eine wirtschaftliche Synthese der Gliptine. Zudem sind diese in hoher Ausbeute und Enentiomerenüberschuss erzielbar.

Die Dehydratisierung in Schritt b) erfolgt jeweils vorzugsweise unter Verwendung eines Chemokatalysators, insbesondere in Gegenwart eines Übergangsmetall-Katalysators, beispielsweise eines Cu(II)-, Zn(II)-, Co(II)- oder Ni(II)-Katalysators. Hierbei sind Cu(II)-basierte Chemokatalysatoren wie Kupfer(II)acetat besonders bevorzugt. Bevorzugt liegt der Stoffmengenanteil des Katalysators im Bereich von ≥ 0,1 mol% bis ≤ 25 mol%, vorzugsweise im Bereich von ≥ 1 mol% bis ≤ 10 mol%, bevorzugt im Bereich von von ≥ 2 mol% bis ≤ 5 mol%, bezogen auf die Stoffmenge des Substrats.

Vorzugsweise führt man die Kondensation des Aldehyds mit Hydroxylamin in Schritt a) in wässriger Lösung, insbesondere in einer Mischung aus Wasser und Alkohol durch. Bevorzugte Alkohole sind ausgewählt aus der Gruppe umfassend Methanol, Ethanol, Isopropanol, *n*-Propanol, *n*-Butanol, *tert*-Butanol, Phenol und/oder Mischungen davon. Der Alkohol ist insbesondere ausgewählt aus *n*-Propanol und/oder Ethanol. Besonders geeignet sind Mischungen von Wasser und Alkohol, beispielsweise Mischungen von Wasser mit Ethanol und/oder n-Propanol. Für die Dehydratisierung des Aldoxims zum Nitril in Schritt b) sind insbesondere organische Lösungsmittel verwendbar. Vorzugsweise führt man die Dehydratisierung des Aldoxims zum α-Aminonitril in einem Lösungsmittel ausgewählt aus Dichlormethan, Methyl-*tert*-butylether, Ethylacetat, Tetrahydrofuran, 2-Methyltetrahydrofuran, Toluol, Acetonitril, Propionitril, Butyronitril und/oder deren Mischungen durch. In bevorzugten Ausführungsformen führt man die Dehydratisierung des Aldoxims zum α-Aminonitril in Anwesenheit einer Nitril-Komponente durch. Vorzugsweise ist das Nitril ausgewählt aus der Gruppe umfassend Acetonitril, Propionitril und/oder Butyronitril. Besonders bevorzugt ist Acetonitril. Vorzugsweise liegt die Nitril-Komponente im Bereich von ≥ 10 Äq. bezogen auf das Aldoxim vor. Die Nitril-Komponente kann auch in höherem Anteil beispielsweise von ≥ 15 Äq. bezogen auf das Aldoxim vorliegen. Weiter sind Mischungen eines Nitrils, insbesondere Acetonitril, mit anderen Lösungsmitteln wie Dichlormethan bevorzugt.

Die Kondensation des Aldehyds mit Hydroxylamin in Schritt a) kann bei Umgebungstemperatur erfolgen. Die Dehydratisierung des Aldoxims zum Nitril in Schritt b) erfolgt vorzugsweise bei erhöhten Temperaturen oder unter Rückfluss. Bevorzugt führt man die Dehydratisierung des Aldoxims zum α-Aminonitril in Schritt b) bei einer Temperatur im Bereich von ≥ 20°C bis ≤ 150°C, vorzugsweise im Bereich von ≥ 50°C bis ≤ 100°C, bevorzugt im Bereich von ≥ 80°C bis ≤ 85°C, durch. Es kann vorgesehen sein, dass sich an eine Reaktionszeit von 1 bis 7 oder 8 Stunden bei diesen Temperaturen eine weitere Umsetzungsphase von bis zu 20 Stunden bei Umgebungstemperatur anschließt.

Beispiele, die der Veranschaulichung der vorliegenden Erfindung dienen, sind nachstehend angegeben.

### Allgemeine Angaben

Chemikalien und Substanzen wurden bei der Firma Sigma-Aldrich bzw. anderen kommerziellen Laborchemikalienanbieter erworben und ohne weitere Aufreinigung verwendet.

Umkehrphasen-Hochleistungsflüssigkeitschromatographie (RP-HPLC) wurde auf einer Nucleodur C₁₈ Htec (Macherey-Nagel) ausgeführt, mit einem Eluent aus Wasser/Acetonitril 50:50 (v/v) unter folgenden Bedingungen 1,0 mL/min, 40 °C, 220 nm. Normalphasen-Hochleistungsflüssigkeitschromatographie (NP-HPLC) wurde auf einer Daicel Chiracel AD-H ausgeführt, mit einem Eluent aus CO₂/Isopropanol 95:5 (v/v), unter folgenden Bedingungen 0,75 mL/min, 30 min bis zu einem Verhältnis 90:10, 2,0 mL/min, 30 min, 20°C, 210 nm.

Gaschromatographie (GC) wurde auf einer Lipodex E (Macherey-Nagel) (0,25 mm ID x 25 m Länge, 0,25 µm film) mit 120 °C Start-Temperatur (35 min), 20 °C/min Temperatur-Rampe und 180 °C End-Temperatur oder CP-Chirasil-Dex CB (Agilent) (0,32 mm ID x 25 m Länge, 0,25 µm film), 160 °C Start-Temperatur (7 min), 2 °C/min Temperatur-Rampe, 180 °C End-Temperatur ausgeführt.

### Allgemeine Arbeitsvorschrift für die Herstellung chiraler N-Acyl-α-aminonitrile aus Aldehyden

Schritt a) Kondensation des Aldehyds mit Hydroxylamin:
Hydroxylamin-Hydrochlorid (1,5 Äq.) und Natriumcarbonat (1,5 Äq.) wurden bei Raumtemperatur (20±2°C) in einer Mischung aus Wasser und n-Propanol oder Wasser und
Ethanol gelöst. Nach Zugabe des Aldehyds wurde die Reaktionsmischung kräftig gerührt, bis die DC-Reaktionskontrolle (Cyclohexan/Ethylacetat in verschiedenen Zusammensetzungen) einen vollständigen Umsatz anzeigte. Die Reaktionslösung wurde drei Mal mit Ethylacetat (1:1 v/v) extrahiert und die vereinten organischen Phasen wurden mit Wasser (1:3 v/v) gewaschen. Nach Trocknen über MgSO₄, Filtrieren und Entfernen des Lösungsmittels wurde das Rohprodukt erhalten, welches bei Bedarf mittels Säulenchromatografie gereinigt wurde.
Das E/Z-Verhältnis des Produkts wurde mit ¹H-NMR-Spektroskopie in CD₂Cl₂ oder in CDCl₃ ermittelt.

Schritt b) Dehydratisierung des Aldoxims zu einem chiralen *N*-Acyl-α-aminonitril unter Kupfer(II)-Katalyse:
Kupfer(II)acetat (10 mol% oder 2 mol%) wurde in Acetonitril gelöst. Nach Zugabe des Aldoxims färbte sich die Reaktionsmischung spontan von cyan nach dunkelgrün. Die Suspension wurde 60 min oder 7 Stunden zum Rückfluss erhitzt. Nach Entfernen des Acetonitrils im Vakuum wurde mit DC-Analyse (Cyclohexan/Ethylacetat in verschiedenen Zusammensetzungen) ein vollständiger Umsatz festgestellt. Das Rohprodukt, welches ein Äquivalent Acetamid enthielt, wurde in Cyclohexan/Ethylacetat (2:1 v/v) gelöst und über eine kurze Kieselgelsäule (4 cm) filtriert, um Acetamid und restliche Kupfersalze zu entfernen.
Nach Entfernen des Lösungsmittels wurde das gewünschte Nitril erhalten. Um die absolute Konfiguration zu ermitteln, wurde das Produkt mit chiraler HPLC oder chiraler GC analysiert.
Der Umsatz zum Nitril wurde alternativ zur ¹H-NMR-Spektroskopie auch über RP-HPLC oder GC bestimmt.

### Beispiel 1

### Herstellung von (S)-N-Boc-Pyrrolidincarbonitril

### 1a) Herstellung von E/Z-N-Boc-l-Prolinaldoxim

Die Synthese erfolgte analog zur allgemeinen Arbeitsvorschrift wie zu Schritt a) beschrieben. 104 mg Hydroxylamin-Hydrochlorid (1,50 mmol) und 159 mg Natriumcarbonat (1,50 mmol) wurden bei Raumtemperatur in 3 mL Wasser und 2 mL Ethanol gelöst. Nach Zugabe von 199 mg N-Boc-l-Prolinal (1.00 mmol) wurde die Lösung 20 Stunden bei Raumtemperatur gerührt, bis die DC-Reaktionskontrolle vollständigen Umsatz anzeigte. Durch Aufarbeitung wurde ein farbloses Öl erhalten. Das Rohprodukt wurde mittels Säulenchromatografie (Cyclohexan/Ethylacetat 3:1, v/v) aufgereinigt. Nach Entfernen des Lösungsmittels bei 40 °C im Vakuum wurde das Produkt mit einem *E*/*Z*-Verhältnis von 65:35 als farbloses Öl erhalten. Die E- und Z-Isomere konnten nicht getrennt werden. Die Isomere wurden mittels ¹H-NMR-Spektroskopie und GC bestätigt. Die Ausbeute an *E*/*Z*-*N*-Boc-l-Prolinaldoxim betrug 143 mg (67%).

### 1b) Herstellung von (S)-N-Boc-Pyrrolidincarbonitril

Die Synthese erfolgte analog zur allgemeinen Arbeitsvorschrift wie zu Schritt b) beschrieben. Zu einer Lösung von 123 mg *E*/*Z*-*N*-Boc-l-Prolinaldoxim (570 µmol) in 7 ml Acetonitril wurden 2,73 mg Kupfer(II)acetat (15,0 µmol) gegeben. Die Reaktionsmischung wurde 7 Stunden zum Rückfluss erhitzt und anschließend 16 Stunden bei Raumtemperatur gerührt. Nach Aufarbeitung wurde das Produkt als farbloses Öl mit einem Enantiomerenüberschuss von 97% erhalten. Die Ausbeute an (*S*)-*N*-Boc-Pyrrolidincarbonitril betrug 97 mg (86%).

### Beispiel 2

### Herstellung von (R)-N-Boc-Pyrrolidincarbonitril

### 2a) Herstellung von E/Z-N-Boc-d-Prolinaldoxim

Die Synthese erfolgte analog zur allgemeinen Arbeitsvorschrift wie zu Schritt a) beschrieben. 104 mg Hydroxylamin-Hydrochlorid (1,5 mmol) und 159 mg Natriumcarbonat (1,5 mmol) wurden bei Raumtemperatur in 3 mL Wasser und 2 mL Ethanol gelöst. Nach Zugabe von 199 mg N-Boc-d-Prolinal (1,0 mmol) wurde die Lösung 24 Stunden bei Raumtemperatur gerührt, bis die DC-Reaktionskontrolle vollständigen Umsatz anzeigte. Durch Aufarbeitung wurde ein farbloses Öl erhalten. Das Rohprodukt wurde mittels Säulenchromatografie (Cyclohexan/Ethylacetat 2:1, v/v) aufgereinigt. Nach Entfernen des Lösungsmittels bei 40 °C im Vakuum wurde das Produkt mit einem E/Z-Verhältnis von 72:28 als farbloses Öl erhalten. Die E- und Z-Isomere konnten nicht getrennt werden. Die Isomere wurden mittels ¹H-NMR-Spektroskopie und GC bestätigt. Die Ausbeute an *E*/*Z*-*N*-Boc-d-Prolinaldoxim betrug 177 mg (81%).

### 2b) Herstellung von (R)-N-Boc-Pyrrolidincarbonitril

Die Synthese erfolgte analog zur allgemeinen Arbeitsvorschrift wie zu Schritt b) beschrieben. Zu einer Lösung von 161 mg *E*/*Z*-*N*-Boc-d-Prolinaldoxim (750 µmol) in 7 ml Acetonitril wurden 2,73 mg Kupfer(II)acetat (15,0 µmol) gegeben. Die Reaktionsmischung wurde 7 Stunden zum Rückfluss erhitzt und anschließend 16 Stunden bei Raumtemperatur gerührt. Nach Aufarbeitung wurde das Produkt als farbloses Öl mit einem Enantiomerenüberschuss von 99% erhalten. Die Ausbeute an (*R*)-*N*-Boc-Pyrrolidincarbonitril betrug 130 mg (88%).

(*R*)-*N*-Boc-Pyrrolidincarbonitril, welches als Nitril-Produkt in der Synthese von Vildagliptin verwendbar ist, wurde mit einem Enantiomerenüberschuss von 99% erhalten. Die Synthese zeigt damit die Robustheit des Verfahrens gegenüber einer etwaige Racemisierung.

### Beispiel 3

### Herstellung von (R)-N-Boc-Phenylalaninnitril

### 3 a) Herstellung von E/Z-N-Boc-d-phenylalaninaloxim

Die Synthese erfolgte analog zur allgemeinen Arbeitsvorschrift wie zu Schritt a) beschrieben. 146 mg Hydroxylaminhydrochlorid (2,11 mmol) und 223 mg Natriumcarbonat (2,11 mmol) wurden in 5 mL Wasser und 5 mL 1-Propanol bei Raumtemperatur gelöst. Nach Zugabe von 350 mg *N*-Boc-d-phenylalaninal (1,40 mmol) wurde die Lösung für 18 Stunden gerührt und vollständiger Umsatz per DC-Kontrolle betätigt. Die Aufarbeitung lieferte ein Gemisch aus E/Z-Isomeren des Produkts als farblosen Feststoff.

Die Isomere wurden per Säulenchromatographie (Cyclohexan/Ethylacetat 3:1, v/v) getrennt, vom Solvens bei Raumtemperatur befreit und als farblose Feststoffe erhalten. Die Isomere E-N-Boc-d-phenylalaninaloxim und *Z*-*N*-Boc-d-phenylalaninaloxim wurden mittels ¹H-NMR-Spektroskopie bestätigt. Die Ausbeute an E-N-Boc-d-phenylalaninaloxim betrug 200 mg (54%) und die Ausbeute an *Z*-*N*-Boc-d-phenylalaninaloxim betrug 142 mg (38%).

### 3 b) Herstellung von (R)-N-Boc-Phenylalaninnitril

Die Synthese erfolgte analog zur allgemeinen Arbeitsvorschrift wie zu Schritt b) beschrieben. 10,3 mg Kupfer(II)acetat (56,7 µmol) wurden in 1,5 mL Acetonitril suspendiert. 150 mg aus Schritt a) erhaltenes *E*/*Z*-*N*-Boc-d-phenylalaninaloxim (567 µmol) wurde hinzugefügt und das Reaktionsgemisch für 60 min zum Rückfluss erhitzt. Die Aufarbeitung

(Cyclohexan/Ethylacetat 2:1, v/v) lieferte das Produkt als farblosen Feststoff. Um die Retention der absoluten Konfiguration zu bestimmen, wurden Messungen per chiraler HPLC durchgeführt. Die Retentionszeit per RP-HPLC betrug Rₜ = 9,0 min und die Retentionszeit per NP-HPLC betrug Rₜ = 23,3 min. Der Umsatz der Reaktion wurde mit RP-HPLC bestimmt. Die Ausbeute an (*R*)-*N*-Boc-Phenylalaninnitril betrug 116 mg (83%).

### Beispiel 4

### Herstellung von (S)-N-Boc-Phenylalaninnitril

### 4a) Herstellung von E/Z-N-Boc-l-phenylalaninaloxim

Die Synthese erfolgte analog zur allgemeinen Arbeitsvorschrift wie zu Schritt a) beschrieben. Die Synthese wurde nach SV1 durchgeführt. 100 mg Hydroxylaminhydrochlorid (1,43 mmol) und 152 mg Natriumcarbonat (1,43 mmol) wurden in 5 mL Wasser und 5 mL 1-Propanol bei Raumtemperatur gelöst. Nach Zugabe von 238 mg *N*-Boc-1-phenylalaninal (955 µmol) wurde die Lösung für 18 Stunden gerührt und vollständiger Umsatz per DC-Kontrolle betätigt. Die Aufarbeitung lieferte ein Gemisch aus E/Z-Isomeren des Produkts als farblosen Feststoff. Die Isomere wurden mittels ¹H-NMR-Spektroskopie bestätigt. Die Ausbeute an (*E*/*Z-N-*Boc-1-phenylalaninaloxim betrug 212 mg (84%).

### 4b) Herstellung von (S)-N-Boc-Phenylalaninnitril

Die Synthese erfolgte analog zur allgemeinen Arbeitsvorschrift wie zu Schritt b) beschrieben. 7,3 mg Kupfer(II)acetat (40,2 µmol) wurden in 1,0 mL Acetonitril suspendiert. 85,0 mg aus Schritt a) erhaltenes *E*/Z-*N*-Boc-l-phenylalaninaloxim (322 µmol) wurden hinzugefügt und das Reaktionsgemisch für 60 min zum Rückfluss erhitzt. Die Aufarbeitung (Cyclohexan/Ethylacetat 2:1, v/v) lieferte das Produkt als farblosen Feststoff. Um die Retention der absoluten Konfiguration zu bestimmen, wurden Messungen per chiraler HPLC durchgeführt. Die Retentionszeit per RP-HPLC betrug Rₜ = 9,0 min und die Retentionszeit per NP-HPLC betrug Rₜ = 20,9 min. Der Umsatz der Reaktion wurde mit RP-HPLC bestimmt. Die Ausbeute an (*S*)-*N*-Boc-Phenylalaninnitril betrug 73 mg (92%).

### Beispiel 5

Untersuchung der Reaktionsparameter der Cu(II)-katalysierten Synthese von (S)-N-Boc-Pyrrolidincarbonitril

Die Cu(II)-katalysierte Synthese von (*S*)-*N*-Boc-Pyrrolidincarbonitril durch Dehydratisieren von *E*/*Z*-*N*-Boc-l-Prolinaldoxim wurde durchgeführt wie unter Beispiel 1b) und der allgemeinen Vorschrift für Schritt b) beschrieben wurde, wobei abweichend jeweils die Menge an Acetonitril und Kupfer(II)acetat variiert bzw. CH₂Cl₂ als Co-Solvenz zugesetzt wurde.

Die Ergebnisse der Dehydratisierungen sind in der folgenden Tabelle 1 zusammengefasst:

**Tabelle 1**

| Eintrag | Menge CH₃CN | Solvens-Zusatz | Menge Cu(OAc)₂ | Reaktionszeit | Umsatz |
|---|---|---|---|---|---|
| 1 | > 100 Äquiv. | / | 10 mol% | 7 h bei 80 °C + 16 h bei 20 °C | quantitativ |
| 2 | > 100 Äquiv. | / | 2 mol% | 7 h bei 80 °C + 16 h bei 20 °C | quantitativ |
| 3 | 10 Äquiv. | CH₂Cl₂ > 25 Äquiv. | 2 mol% | 7h bei 80 °C+ 16 h bei 20 °C | quantitativ |

Wie man der Tabelle 1 entnimmt, wurde bei einem Stoffmengenanteil an Cu(OAc)₂ als Katalysator in einem Bereich von 2 bis 10 mol% bezogen auf das Substrat eine vollständige Umsetzung erzielt. Zudem ließ sich durch die Verwendung von Dichlormethan als Co-Solvenz die Menge des eingesetzten Acetonitrils bei ebenfalls quantitativer Umsetzung reduzieren.

## Patentansprüche

1. Verfahren zur Herstellung eines *N*-Acyl- oder *N*-Sulfonyl-α-aminonitrils, umfassend die folgenden Schritte:
a) Kondensation eines *N*-Acyl- oder *N*-Sulfonyl-α-aminoaldehyds gemäß der allgemeinen Formel (I) mit Hydroxylamin zu einem Aldoxim gemäß der allgemeinen Formel (II), und
b) Dehydratisierung des in Schritt a) erhaltenen Aldoxims zu einem *N*-Acyl- oder *N-*Sulfonyl-α-aminonitril gemäß der allgemeinen Formel (III): 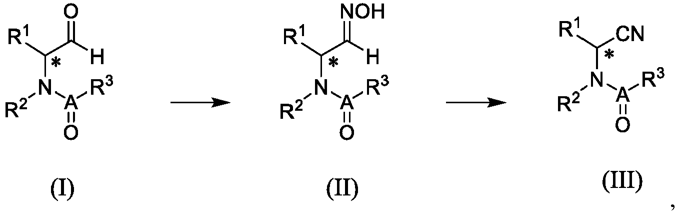 worin:
A ist C oder S=O;
R¹ ist ausgewählt aus der Gruppe umfassend verzweigtes oder unverzweigtes C₁-C₂₀-Alkyl, C₆-C₁₀-Aryl, C₆-C₁₆-Heteroaryl, C₇-C₁₆-Arylalkyl und/oder C₆-C₁₆-Heteroarylalkyl, wobei diese unsubstituiert oder einfach oder mehrfach substituiert mit wenigstens einem Substituenten ausgewählt aus der Gruppe umfassend OH, NH₂, NHR⁴, NR⁴₂, C₁₋₄-Alkyl, C₇-C₁₆-Arylalkyl, C₆-C₁₆-Heteroarylalkyl, Carbonylsauerstoff und/oder C₁₋₄-Alkoxy sind;
R² ist ausgewählt aus der Gruppe umfassend H, verzweigtes oder unverzweigtes C₁-C₂₀-Alkyl, C₆-C₁₀-Aryl, C₆-C₁₆-Heteroaryl, C₇-C₁₆-Arylalkyl und/oder C₆-C₁₆-Heteroarylalkyl, wobei diese unsubstituiert oder einfach oder mehrfach substituiert mit wenigstens einem Substituenten ausgewählt aus der Gruppe umfassend OH, NH₂, NHR⁴, NR⁴₂, C₁₋₄-Alkyl, C₇-C₁₆-Arylalkyl, C₆-C₁₆-Heteroarylalkyl, Carbonylsauerstoff und/oder C₁₋₄-Alkoxy sind; oder
R¹ und R² bilden gemeinsam einen gesättigten 5- oder 6-gliedrigen Ring oder ein bicyclisches Ringsystem aus, wobei diese wenigstens ein weiteres Heteroatom ausgewählt aus N, O und/oder S enthalten können und/oder diese einfach oder mehrfach mit wenigstens einem Substituenten ausgewählt aus der Gruppe umfassend OH, NH₂, NHR⁴, NR⁴₂, C₁₋₄-Alkyl, Carbonylsauerstoff und/oder C₁₋₄-Alkoxy substituiert sein können;
R³ ist ausgewählt aus der Gruppe umfassend H, C₁₋₆-Alkoxy und/oder C₁₋₆-Alkyl, wobei diese einfach oder mehrfach substituiert sind mit wenigstens einem Substituenten ausgewählt aus der Gruppe umfassend OH, OR⁴, NH₂, NHR⁴, NR⁴₂, NHY und/oder Halogen; oder ist ausgewählt aus der Gruppe der Strukturelemente (IV), (V) und (VI) wie folgt: 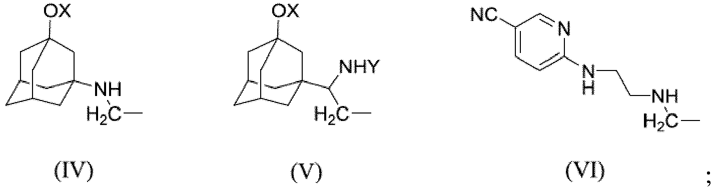
R⁴ ist jeweils gleich oder unabhängig voneinander ausgewählt aus der Gruppe umfassend C₁-C₁₈-Alkyl oder C₁-C₁₈-Acyl;
X, Y sind jeweils gleich oder unabhängig voneinander H oder eine Schutzgruppe insbesondere ausgewählt aus *tert*-Butyloxycarbonyl (Boc), Benzyloxycarbonyl, Acetyl, Silyl, p-Tolyl, Trifluormethyl und/oder Sulfonyl.

2. Verfahren nach Anspruch 1, **dadurch gekennzeichnet, dass** man in Schritt a) ein enantiomerenangereichertes oder enantiomerenreines *N*-Acyl- oder *N*-Sulfonyl-α-aminoaldehyd einsetzt, wobei dessen absolute Konfiguration bei der Umwandlung zum *N*-Acyl- oder *N*-Sulfonyl-α-aminonitril erhalten oder im Wesentlichen erhalten bleibt.

3. Verfahren nach Anspruch 1 oder 2, **dadurch gekennzeichnet, dass** die Substituenten R¹, R² und R³ der Verbindungen gemäß der allgemeinen Formeln (I), (II) und (III) die folgenden sind:
A ist C;
R¹ ist ausgewählt aus der Gruppe umfassend Benzyl und/oder C₁-C₂-Alkyl,
R² ist ausgewählt aus der Gruppe umfassend H, Benzyl und/oder C₁-C₂-Alkyl, oder
R¹ und R² bilden gemeinsam einen gesättigten 5-gliedrigen Ring oder Bicyclo[3.1.0]hexan aus, und
R³ ist ausgewählt aus der Gruppe umfassend H, *tert*-Butoxy, Chloromethyl, Strukturelement (IV), (V) und/oder (VI).

4. Verfahren nach einem der vorherigen Ansprüche, **dadurch gekennzeichnet, dass** man in Schritt b) die Dehydratisierung des Aldoxims zum *N*-Acyl- oder *N*-Sulfonyl-α-Aminonitril in Gegenwart eines Übergangsmetall-Katalysators, insbesondere eines Cu(II)-, Zn(II)-, Co(II)- oder Ni(II)-Katalysators, durchführt.

5. Verfahren nach einem der vorherigen Ansprüche, **dadurch gekennzeichnet, dass** der Stoffmengenanteil des Katalysators im Bereich von ≥ 0,1 mol% bis ≤ 25 mol%, vorzugsweise im Bereich von ≥ 1 mol% bis ≤ 10 mol%, bevorzugt im Bereich von von ≥ 2 mol% bis ≤ 5 mol%, liegt.

6. Verfahren nach einem der vorherigen Ansprüche, **dadurch gekennzeichnet, dass** man die Dehydratisierung des Aldoxims zum *N*-Acyl- oder *N*-Sulfonyl-α-Aminonitril in Schritt b) in Anwesenheit einer Nitril-Komponente vorzugsweise ausgewählt aus der Gruppe umfassend Acetonitril, Propionitril und/oder Butyronitril durchführt, wobei die Nitril-Komponente vorzugsweise im Bereich von ≥ 10 Äq. bezogen auf das Aldoxim vorliegt.

7. Verfahren nach einem der vorherigen Ansprüche, **dadurch gekennzeichnet, dass** man die Dehydratisierung des Aldoxims zum *N*-Acyl- oder *N*-Sulfonyl-α-Aminonitril in Schritt b) bei einer Temperatur im Bereich von ≥ 20°C bis ≤ 150°C, vorzugsweise im Bereich von ≥ 50°C bis ≤ 100°C, bevorzugt im Bereich von ≥ 80°C bis ≤ 85°C, durchführt.

8. Verfahren nach Anspruch 1 zur Herstellung von Vildagliptin oder dessen Salzen, umfassend die folgenden Schritte:
a) Kondensieren eines Aldehyds der Formel (1) mit Hydroxylamin zu einem Aldoxim der Formel (2): 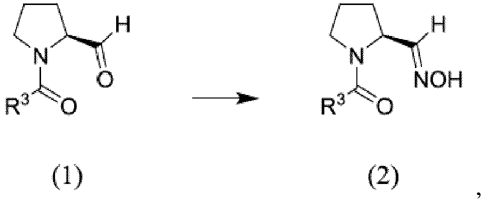 worin
R³ ist -CH₂- substituiert mit einem Substituenten ausgewählt aus der Gruppe umfassend OH, OR⁴, NH₂, NHR⁴, NR⁴₂, NHY und/oder Halogen oder Strukturelement (IV) wie folgt: 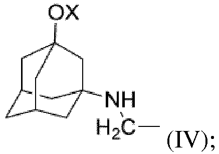
X, Y sind jeweils gleich oder unabhängig voneinander H oder eine Schutzgruppe insbesondere ausgewählt aus *tert*-Butyloxycarbonyl (Boc), Benzyloxycarbonyl, Acetyl, Silyl, p-Tolyl, Trifluormethyl und/oder Sulfonyl:
R⁴ ist jeweils gleich oder unabhängig voneinander ausgewählt aus der Gruppe umfassend C₁-C₁₈-Alkyl oder C₁-C₁₈-Acyl;
b) Dehydratisierung des in Schritt a) erhaltenen Aldoxims der Formel (2) zu einem N-Acyl-α-aminonitril der Formel (3): 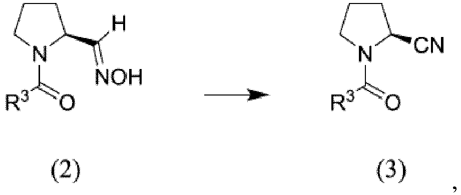
c) optional Umsetzen des *N*-Acyl-α-aminonitrils der Formel (3) mit R³ ist -CH₂-substituiert mit einem Substituenten ausgewählt aus der Gruppe umfassend OH, OR⁴, NH₂, NHR⁴, NR⁴₂, NHY und/oder Halogen mit 1-Aminoadamantan-3-ol oder einem geschützten Derivat der Formel (4) zur Verbindung der Formel (5): 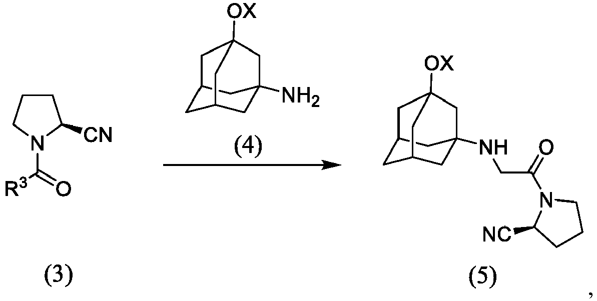 und
d) optional Abspalten der Schutzgruppe X zu Vildagliptin der Formel (6):

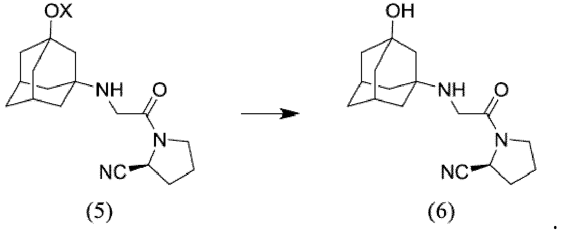

9. Verfahren nach Anspruch 1 zur Herstellung von Saxagliptin oder dessen Salzen, umfassend die folgenden Schritte:
a) Kondensation eines Aldehyds der Formel (7) mit Hydroxylamin zu einem Aldoxim der Formel (8): 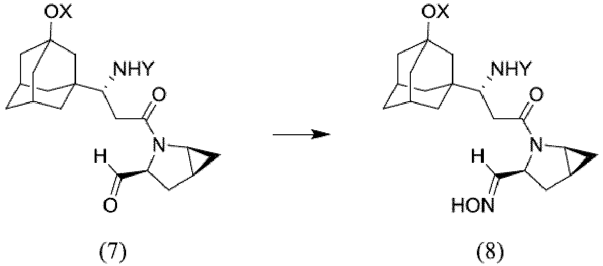 worin:
X, Y sind jeweils gleich oder unabhängig voneinander H oder eine Schutzgruppe insbesondere ausgewählt aus *tert*-Butyloxycarbonyl (Boc), Benzyloxycarbonyl, Acetyl, Silyl, p-Tolyl, Trifluormethyl und/oder Sulfonyl;
b) Dehydratisierung des in Schritt a) erhaltenen Aldoxims der Formel (8) zu einem N-Acyl-α-aminonitril der Formel (9): 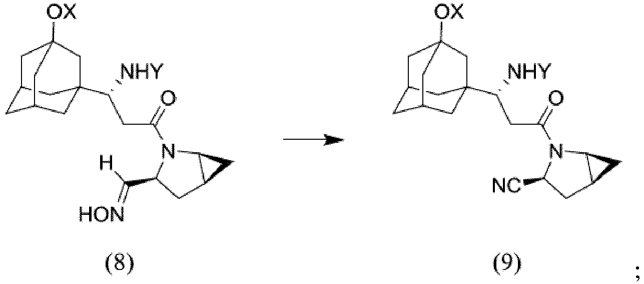
c) optional Abspalten der Schutzgruppen X, Y zu Saxagliptin (10):

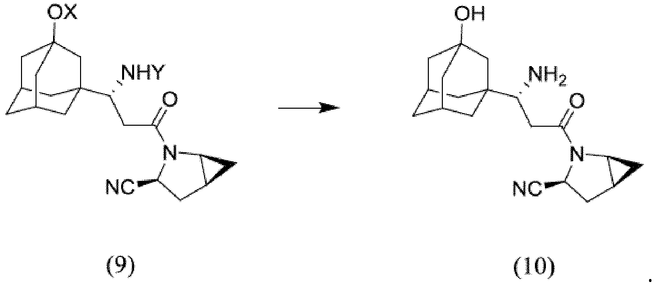

## Claims

1. Method for preparing an *N*-acyl- or *N*-sulfonyl-α-aminonitrile, comprising the following steps:
a) condensation of an *N*-acyl- or *N*-sulfonyl-α-aminoaldehyde according to the general formula (I) with hydroxylamine to give an aldoxime according to the general formula (II), and
b) dehydration of the aldoxime obtained in step a) to give an *N*-acyl- or *N*-sulfonyl-α-aminonitrile according to the general formula (III): 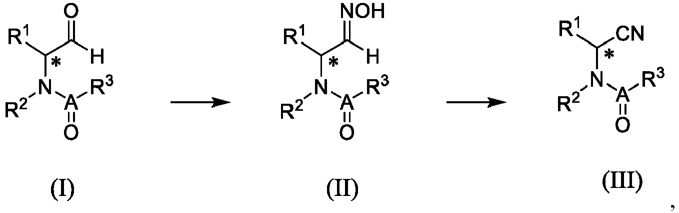 in which:
A is C or S=O;
R¹ is selected from the group comprising branched or unbranched C₁-C₂₀-alkyl, C₆-C₁₀-aryl, C₆-C₁₆-heteroaryl, C₇-C₁₆-arylalkyl and/or C₆-C₁₆-heteroarylalkyl, wherein these are unsubstituted or monosubstituted or polysubstituted by at least one substituent selected from the group comprising OH, NH₂, NHR⁴, NR⁴₂, C₁₋₄-alkyl, C₇-C₁₆-arylalkyl, C₆-C₁₆-heteroarylalkyl, carbonyl oxygen and/or C₁₋₄-alkoxy;
R² is selected from the group comprising H, branched or unbranched C₁-C₂₀-alkyl, C₆-C₁₀-aryl, C₆-C₁₆-heteroaryl, C₇-C₁₆-arylalkyl and/or C₆-C₁₆-heteroarylalkyl, wherein these are unsubstituted or monosubstituted or polysubstituted by at least one substituent selected from the group comprising OH, NH₂, NHR⁴, NR⁴₂, C₁₋₄-alkyl, C₇-C₁₆-arylalkyl, C₆-C₁₆-heteroarylalkyl, carbonyl oxygen and/or C₁₋₄-alkoxy; or
R¹ and R² together form a saturated 5- or 6-membered ring or a bicyclic ring system, wherein these may comprise at least one further heteroatom selected from N, O and/or S and/or these can be monosubstituted or polysubstituted by at least one substituent selected from the group comprising OH, NH₂, NHR⁴, NR⁴₂, C₁₋₄-alkyl, carbonyl oxygen and/or C₁₋₄-alkoxy;
R³ is selected from the group comprising H, C₁₋₆-alkoxy and/or C₁₋₆-alkyl, wherein these are monosubstituted or polysubstituted by at least one substituent selected from the group comprising OH, OR⁴, NH₂, NHR⁴, NR⁴₂, NHY and/or halogen; or is selected from the group of the structural elements (IV), (V) and (VI) as follows: 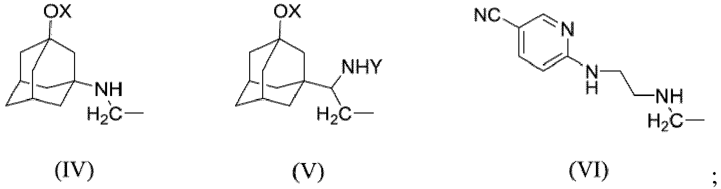
R⁴ is in each case identical or each independently selected from the group comprising C₁-C₁₈-alkyl or C₁-C₁₈-acyl;
X, Y are in each case identical or each independently H or a protecting group, especially selected from *tert*-butyloxycarbonyl (Boc), benzyloxycarbonyl, acetyl, silyl, p-tolyl, trifluoromethyl and/or sulfonyl.

2. Method according to claim 1, **characterized in that** in step a) an enantiomerically enriched or an enantiomerically pure *N*-acyl- or *N*-sulfonyl-α-aminoaldehyde is used, the absolute configuration of which is retained or substantially retained in the conversion to the *N*-acyl- or *N*-sulfonyl-α-aminonitrile.

3. Method according to claim 1 or 2, **characterized in that** the substituents R¹, R² and R³ of the compounds according to the general formulae (I), (II) and (III) are the following:
A is C;
R¹ is selected from the group comprising benzyl and/or C₁-C₂-alkyl,
R² is selected from the group comprising H, benzyl and/or C₁-C₂-alkyl, or
R¹ and R² together form a saturated 5-membered ring or bicyclo[3.1.0]hexane, and
R³ is selected from the group comprising H, *tert*-butoxy, chloromethyl, structural element (IV), (V) and/or (VI).

4. Method according to any of the preceding claims, **characterized in that** in step b) the dehydration of the aldoxime to give the *N*-acyl- or *N*-sulfonyl-α-aminonitrile is carried out in the presence of a transition metal catalyst, especially a Cu(II), Zn(II), Co(II) or Ni(II) catalyst.

5. Method according to any of the preceding claims, **characterized in that** the mole fraction of the catalyst is in the range from ≥ 0.1 mol% to ≤ 25 mol%, preferably in the range from ≥ 1 mol% to ≤ 10 mol%, preferably in the range from ≥ 2 mol% to ≤ 5 mol%.

6. Method according to any of the preceding claims, **characterized in that** the dehydration of the aldoxime to give the *N*-acyl- or *N*-sulfonyl-α-aminonitrile in step b) is carried out in the presence of a nitrile component preferably selected from the group comprising acetonitrile, propionitrile and/or butyronitrile, wherein the nitrile component is preferably present in the range of ≥ 10 eq., based on the aldoxime.

7. Method according to any of the preceding claims, **characterized in that** the dehydration of the aldoxime to give the *N*-acyl- or *N*-sulfonyl-α-aminonitrile in step b) is conducted at a temperature in the range from ≥ 20°C to ≤ 150°C, preferably in the range from ≥ 50°C to ≤ 100°C, preferably in the range from ≥ 80°C to ≤ 85°C.

8. Method according to claim 1 for preparing vildagliptin or salts thereof, comprising the following steps:
a) condensing an aldehyde of the formula (1) with hydroxylamine to give an aldoxime of the formula (2): 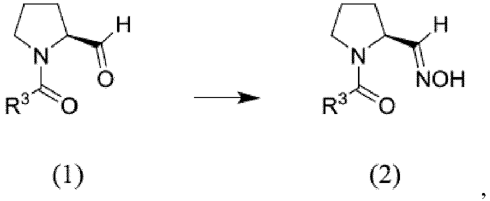 in which
R³ is -CH₂- substituted by a substituent selected from the group comprising OH, OR⁴, NH₂, NHR⁴, NR⁴₂, NHY and/or halogen or structural element (IV) as follows: 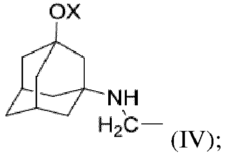
X, Y are identical or each independently H or a protecting group, especially selected from *tert*-butyloxycarbonyl (Boc), benzyloxycarbonyl, acetyl, silyl, p-tolyl, trifluoromethyl and/or sulfonyl:
R⁴ is identical or each independently selected from the group comprising C₁-C₁₈-alkyl or C₁-C₁₈-acyl;
b) dehydration of the aldoxime of the formula (2) obtained in step a) to give an *N*-acyl-α-aminonitrile of the formula (3): 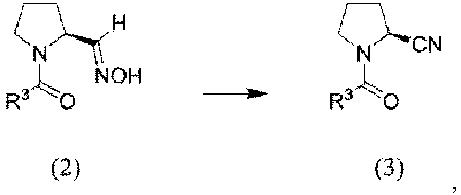
c) optional reaction of the *N*-acyl-α-aminonitrile of the formula (3), where R³ is -CH₂-substituted by a substituent selected from the group comprising OH, OR⁴, NH₂, NHR⁴, NR⁴₂, NHY and/or halogen, with 1-aminoadamantane-3-ol or a protected derivative of the formula (4) to give the compound of the formula (5): 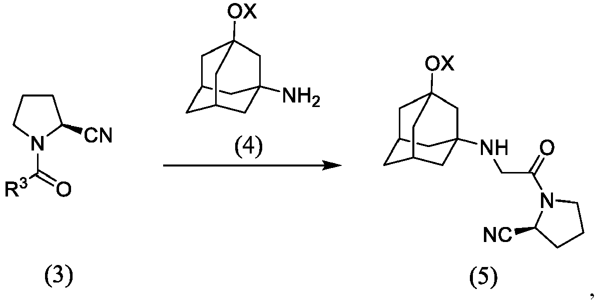 and
d) optional cleavage of the protecting group X to give vildagliptin of the formula (6):

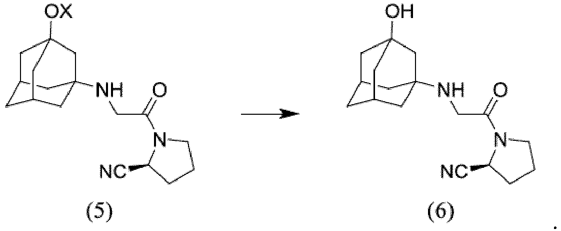

9. Method according to claim 1 for preparing saxagliptin or salts thereof, comprising the following steps:
a) condensation of an aldehyde of the formula (7) with hydroxylamine to give an aldoxime of the formula (8): 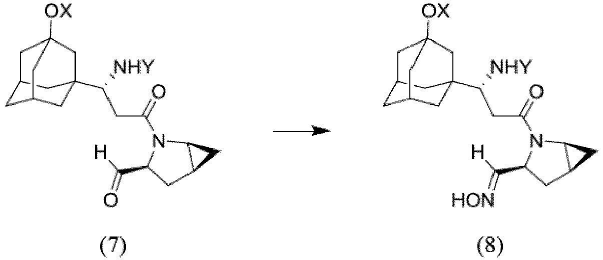 in which:
X, Y are identical or each independently H or a protecting group, especially selected from *tert*-butyloxycarbonyl (Boc), benzyloxycarbonyl, acetyl, silyl, p-tolyl, trifluoromethyl and/or sulfonyl;
b) dehydration of the aldoxime of the formula (8) obtained in step a) to give an *N*-acyl-α-aminonitrile of the formula (9): 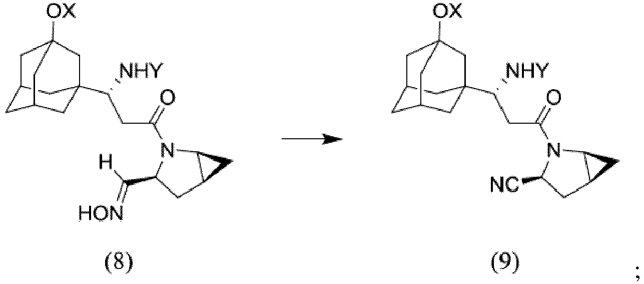
c) optional cleavage of the protecting groups X, Y to give saxagliptin (10):

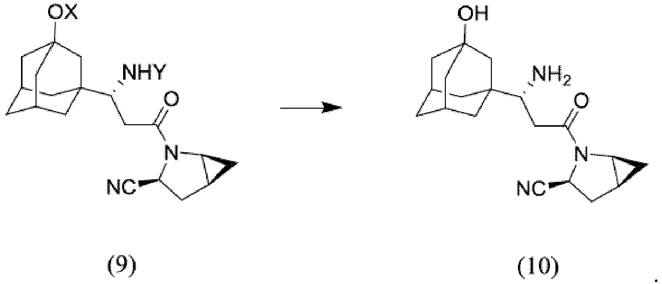

## Revendications

1. Procédé de fabrication d'un *N*-acyl- ou *N*-sulfonyl-α-aminonitrile, comprenant les étapes suivantes :
a) la condensation d'un *N*-acyl- ou *N*-sulfonyl-α-aminoaldéhyde selon la formule générale (I) avec de l'hydroxylamine en un aldoxime selon la formule générale (II), et
b) la déshydratation de l'aldoxime obtenu dans l'étape a) en un *N*-acyl- ou *N*-sulfonyl-α-aminonitrile selon la formule générale (III) : 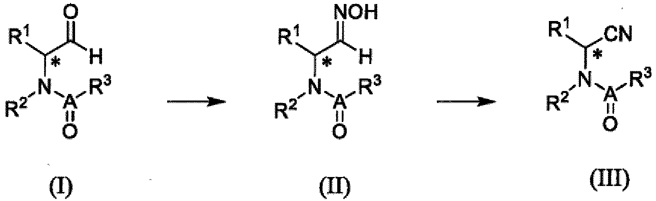 dans lesquelles :
A représente C ou S=O ;
R¹ est choisi dans le groupe comprenant alkyle en C₁-C₂₀, aryle en C₆-C₁₀, hétéroaryle en C₆-C₁₆, arylalkyle en C₇-C₁₆ et/ou hétéroarylalkyle en C₆-C₁₆ ramifiés ou non ramifiés, ceux-ci étant non substitués ou substitués une ou plusieurs fois avec au moins un substituant choisi dans le groupe comprenant OH, NH₂, NHR⁴, NR⁴₂, alkyle en C₁₋₄, arylalkyle en C₇-C₁₆, hétéroarylalkyle en C₆-C₁₆, carbonyl-oxygène et/ou alcoxy en C₁-C₄ ;
R² est choisi dans le groupe comprenant H, alkyle en C₁-C₂₀, aryle en C₆-C₁₀, hétéroaryle en C₆-C₁₆, arylalkyle en C₇-C₁₆ et/ou hétéroarylalkyle en C₆-C₁₆ ramifiés ou non ramifiés, ceux-ci étant non substitués ou substitués une ou plusieurs fois avec au moins un substituant choisi dans le groupe comprenant OH, NH₂, NHR⁴, NR⁴₂, alkyle en C₁₋₄, arylalkyle en C₇-C₁₆, hétéroarylalkyle en C₆-C₁₆, carbonyl-oxygène et/ou alcoxy en C₁-C₄ ; ou
R¹ et R² forment ensemble un cycle saturé à 5 ou 6 chaînons ou un système cyclique bicyclique, ceux-ci pouvant contenir au moins un hétéroatome supplémentaire choisi parmi N, O et/ou S et/ou ceux-ci pouvant être substitués une ou plusieurs fois avec au moins un substituant choisi dans le groupe comprenant OH, NH₂, NHR⁴, NR⁴₂, alkyle en C₁₋₄, carbonyl-oxygène et/ou alcoxy en C₁-C₄ ;
R³ est choisi dans le groupe comprenant H, alcoxy en C₁₋₆ et/ou alkyle en C₁₋₆, ceux-ci étant substitués une ou plusieurs fois avec au moins un substituant choisi dans le groupe comprenant OH, OR⁴, NH₂, NHR⁴, NR⁴₂, NHY et/ou halogène ; ou est choisi dans le groupe des éléments structuraux (IV), (V) et (VI) ci-dessous : 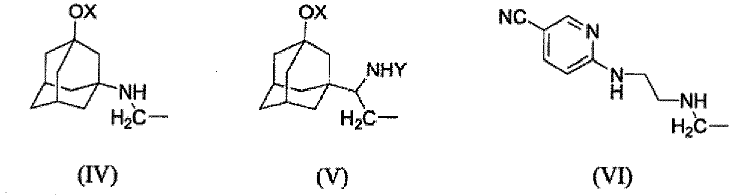
les R⁴ sont respectivement de manière identique ou indépendamment les uns des autres choisis dans le groupe comprenant alkyle en C₁-C₁₈ ou acyle en C₁-C₁₈ ;
X, Y représentent respectivement de manière identique ou indépendamment l'un de l'autre H ou un groupe protecteur notamment choisi parmi tert-butyloxycarbonyle (Boc), benzyloxycarbonyle, acétyle, silyle, p-tolyle, trifluorométhyle et/ou sulfonyle.

2. Procédé selon la revendication 1, **caractérisé en ce que**, dans l'étape a), un *N*-acyl- ou *N*-sulfonyl-α-aminoaldéhyde énantiomériquement enrichi ou énantiomériquement pur est utilisé, dont la configuration absolue est conservée ou essentiellement conservée lors de la transformation en le *N*-acyl- ou N-sulfonyl-α-aminonitrile.

3. Procédé selon la revendication 1 ou 2, **caractérisé en ce que** les substituants R¹, R² et R³ des composés selon les formules générales (I), (II) et (III) sont les suivants :
A représente C ;
R¹ est choisi dans le groupe comprenant benzyle et/ou alkyle en C₁-C₂,
R² est choisi dans le groupe comprenant H, benzyle et/ou alkyle en C₁-C₂,
ou
R¹ et R² forment ensemble un cycle saturé à 5 chaînons ou bicyclo[3.1.0]hexane, et
R³ est choisi dans le groupe comprenant H, *tert-*butoxy, chlorométhyle, l'élément structural (IV), (V) et/ou (VI).

4. Procédé selon l'une quelconque des revendications précédentes, **caractérisé en ce que**, dans l'étape b), la déshydratation de l'aldoxime en le *N*-acyl- ou N-sulfonyl-α-aminonitrile est réalisée en présence d'un catalyseur à base de métal de transition, notamment d'un catalyseur à base de Cu(II), Zn(II), Co(II) ou Ni (II) .

5. Procédé selon l'une quelconque des revendications précédentes, **caractérisé en ce que** la proportion de quantité de matière du catalyseur se situe dans la plage allant de ≥ 0,1 % en moles à ≤ 25 % en moles, de préférence dans la plage allant de ≥ 1 % en moles à ≤ 10 % en moles, de préférence dans la plage allant de ≥ 2 % en moles à ≤ 5 % en moles.

6. Procédé selon l'une quelconque des revendications précédentes, **caractérisé en ce que** la déshydratation de l'aldoxime en le *N*-acyl- ou *N*-sulfonyl-α-aminonitrile dans l'étape b) est réalisée en présence d'un composant nitrile, de préférence choisi dans le groupe comprenant l'acétonitrile, le propionitrile et/ou le butyronitrile, le composant nitrile étant de préférence présent dans la plage de ≥ 10 éq. par rapport à l'aldoxime.

7. Procédé selon l'une quelconque des revendications précédentes, **caractérisé en ce que** la déshydratation de l'aldoxime en le *N*-acyl- ou *N*-sulfonyl-α-aminonitrile dans l'étape b) est réalisée à une température dans la plage allant de ≥ 20 °C à ≤ 150 °C, de préférence dans la plage allant de ≥ 50 °C à ≤ 100 °C, préférentiellement dans la plage allant de ≥ 80 °C à ≤ 85 °C.

8. Procédé selon la revendication 1 pour la fabrication de vildagliptine ou ses sels, comprenant les étapes suivantes :
a) la condensation d'un aldéhyde de la formule (1) avec de l'hydroxylamine en un aldoxime de la formule (2) : 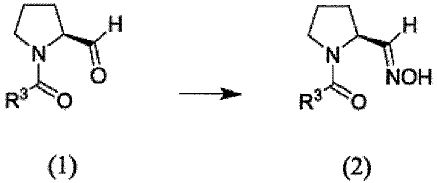 dans lesquelles
R³ représente -CH₂- substitué avec un substiuant choisi dans le groupe comprenant OH, OR⁴, NH₂, NHR⁴, NR⁴₂, NHY et/ou halogène ou l'élément structural (IV) ci-dessous : 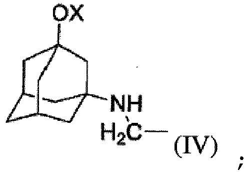
X, Y représentent respectivement de manière identique ou indépendamment l'un de l'autre H ou un groupe protecteur notamment choisi parmi *tert-*butyloxycarbonyle (Boc), benzyloxycarbonyle, acétyle, silyle, p-tolyle, trifluorométhyle et/ou sulfonyle ;
les R⁴ sont respectivement de manière identique ou indépendamment les uns des autres choisis dans le groupe comprenant alkyle en C₁-C₁₈ ou acyle en C₁-C₁₈ ;
b) la déshydratation de l'aldoxime de la formule (2) obtenu dans l'étape a) en un *N*-acyl-α-aminonitrile de la formule (3) : 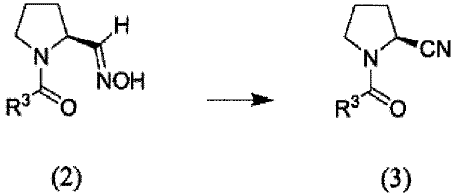
c) éventuellement la mise en réaction du *N*-acyl-α-aminonitrile de la formule (3) dans laquelle R³ représente -CH₂- substitué avec un substituant choisi dans le groupe comprenant OH, OR⁴, NH₂, NHR⁴, NR⁴₂, NHY et/ou halogène avec du 1-aminoadamantan-3-ol ou un dérivé protégé de la formule (4) pour former le composé de la formule (5) : 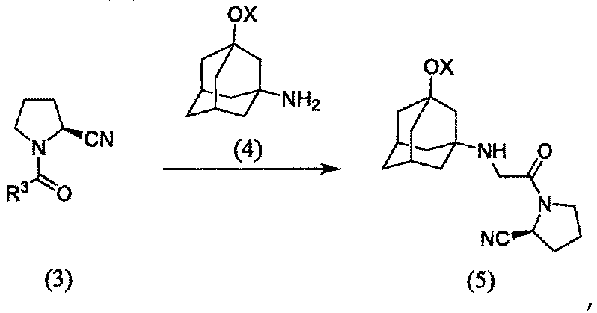 et
d) éventuellement le clivage du groupe protecteur X pour former la vildagliptine de la formule (6) :

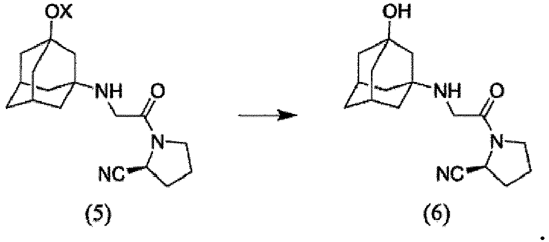

9. Procédé selon la revendication 1 pour la fabrication de saxagliptine ou ses sels, comprenant les étapes suivantes :
a) la condensation d'un aldéhyde de la formule (7) avec de l'hydroxylamine en un aldoxime de la formule (8) : 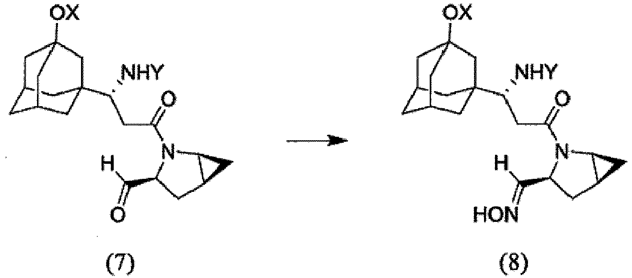 dans lesquelles :
X, Y représentent respectivement de manière identique ou indépendamment l'un de l'autre H ou un groupe protecteur notamment choisi parmi *tert-*butyloxycarbonyle (Boc), benzyloxycarbonyle, acétyle, silyle, p-tolyle, trifluorométhyle et/ou sulfonyle ;
b) la déshydratation de l'aldoxime de la formule (8) obtenu dans l'étape a) en un *N*-acyl-α-aminonitrile de la formule (9) : 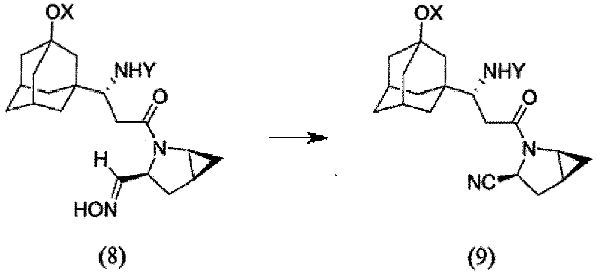
c) éventuellement le clivage des groupes protecteurs X, Y pour former la saxagliptine (10) :
